# EUROPÄISCHE PATENTSCHRIFT

(11) **EP 1 572 142 B1**
(45) Veröffentlichungstag und Bekanntmachung des Hinweises auf die Patenterteilung: **10.02.2010**
(21) Anmeldenummer: 03789261.9
(22) Anmeldetag: 11.12.2003
(51) Int. Cl.: A61Q 11/00, A61K 8/73, A61K 8/19

(54) **KOMPOSITMATERIALIEN AUS CALCIUMVERBINDUNGEN UND GLUCURONSÄUREHALTIGEN POLYSACCHARIDEN**
COMPOSITE MATERIALS MADE OF CALCIUM COMPOUNDS AND POLYSACCHARIDES CONTAINING GLUCURONIC ACID
MATIERES COMPOSITES CONSTITUEES DE COMPOSES DE CALCIUM ET DE POLYSACCHARIDES CONTENANT DE L'ACIDE GLUCURONIQUE

(30) Priorität: 20.12.2002 DE 10260958
(43) Veröffentlichungstag der Anmeldung: 14.09.2005
(73) Patentinhaber: Henkel AG & Co. KGaA, 40589 Düsseldorf (DE)
(72) Erfinder: SCHECHNER, Gallus, 64285 Darmstadt (DE); BRAUNBARTH, Carola, 64289 Darmstadt (DE); FRANKE, Holger, 65462 Ginsheim-Gustavsburg (DE); POTH, Tilo, 69469 Weinheim (DE)
(86) Internationale Anmeldenummer: PCT/EP2003/014183
(87) Internationale Veröffentlichungsnummer: WO 2004/058207

(56) Entgegenhaltungen:
- EP-A- 0 296 078
- WO-A-01/01930
- WO-A-97/16160
- WO-A-02/070030
- DE-A- 19 811 900
- FR-A- 2 585 576
- US-A- 4 299 790
- US-A1- 2003 082 808
- BAKOS D ET AL: "Hydroxyapatite-collagen-hyaluronic acid composite" BIOMATERIALS, ELSEVIER SCIENCE PUBLISHERS BV., BARKING, GB, Bd. 20, Nr. 2, Januar 1999 (1999-01), Seiten 191-195, XP004168893 ISSN: 0142-9612
- DATABASE EMBASE [Online] ELSEVIER SCIENCE PUBLISHERS, AMSTERDAM, NL; 1999 Database accession no. emb 1996050850 XP002277493 & VERRIER ET AL.: "Human osteoprogenitor responses to orthopeadic implant : Mechanism of cell attachment and cell adhesion" JOURNAL OF MATERIALS SCIENCE : MATERIALS IN MEDICINE, Bd. 7, Nr. 1, 1999, Seiten 46-51,

## Beschreibung

Die Erfindung betrifft. Kompositmaterialien aus schwer wasserlöslichen Calciumsalzen und Glucuronsäure- haltigen Polysacchariden, die sich aufgrund ihrer Zusammensetzung und Feinstruktur besonders zur Förderung der Wiederherstellung von Knochen und Zahnschmelz eignen.

Phosphatsalze des Calciums werden seit langem sowohl als Abrasivkomponenten als auch zur Förderung der Remineralisierung des Zahnschmelzes den Rezepturen von Zahnreinigungsmitteln und Zahnpflegemitteln zugesetzt. Dies gilt insbesondere für Hydroxylapatit und Fluorapatit sowie für amorphe Calciumphosphate und für Brushit (Dicalciumphosphat-dihydrat). Auch Calciumfluorid ist als Bestandteil von Zahnreinigungsmitteln und als Komponente zur Festigung des Zahnschmelzes und zur Kariesprophylaxe mehrfach beschrieben worden.

Die Verfügbarkeit von Calcium-Verbindungen für die erwünschte Remineralisierung hängt ganz entscheidend von der Teilchengröße dieser in Wasser schwerlöslichen und in den Zahnpflegemitteln dispergierten Komponenten ab. Man hat daher vorgeschlagen, diese schwerlöslichen Calciumsalze in feinster Verteilung einzusetzen.

Der Zahnschmelz sowie das Stützgewebe der Knochen bestehen überwiegend aus dem Mineral Hydroxylapatit. Im biologischen Entstehungsprozeß lagert sich Hydroxylapatit in geordneter Weise an die Proteinmatrix im Knochen oder Zahn an, die überwiegend aus Kollagen besteht. Die Ausbildung der harten und belastungsfähigen mineralischen Strukturen wird dabei durch die sogenannten Matrixproteine gesteuert, welche neben Kollagen durch weitere Proteine gebildet werden, die sich an das .Kollagen anlagern und so einen strukturierten Mineralisierungsprozeß, der auch als Biomineralisation bezeichnet wird, bewirken.

Bei der Wiederherstellung von Knochenmaterial spielen sogenannte Knochenersatzmittel, welche den natürlichen Biomineralisationsprozeß fördern, eine wichtige Rolle. Derartige Mittel werden auch benötigt zur Beschichtung von Implantaten, um stoffschlüssige Verbindungen zwischen Knochen und Implantat zu erreichen, mit denen auch Zugkräfte übertragen werden können. Von besonderer Bedeutung sind hier Beschichtung mit einer hohen Bioaktivität, die zu einer wirksamen Verbundosteogenese führen. Nach dem Stand der Technik, wie ihn z. B. G. Willmann in Mat.-wiss. u. Werkstofftech. 30 (1999), 317 beschreibt, wird in der Regel Hydroxylapatit auf Implantate aufgebracht. Nachteilig an dieser Vorgehensweise ist neben der oft unzureichenden Beschleunigung des Biomineralisationsprozesses das Abplatzen der Hydroxylapatit-Schichten und ihre unbefriedigende chemische Stabilität.

Aus dem Stand der Technik (bspw. der WO01/01930) bekannte proteinhaltige Kompositmaterialien enthalten Proteine, die vorwiegend aus bovinem Material gewonnen werden. Besonders in der Kosmetik besteht aufgrund der BSE-Krise ein zunehmender Wunsch nach Produkten, die frei von Inhaltsstoffen bovinen Ursprungs sind. Es besteht daher ein Bedürfnis auch nach alternativen, vergleichbar gut wirkenden Kompositmaterialien, welche keine Komponenten tierischen Ursprungs enthalten.

Weiterhin weisen die aus dem Stand der Technik bekannten Kompositmaterialien in einigen Lösungsmittel, insbesondere in Wasser eine ungünstige Dispergierbarkeit auf und sind in die für ihre gewerbliche Anwendung erforderlichen Formulierungen schlecht einarbeitbar oder weisen eine unbefriedigende Dispersionsstabilität in den zum Gebrauch kommenden Zubereitungen auf.

So sedimentiert beispielsweise eine 2-prozentige Dispersion eines Apatit-Gelatine-Komposit ohne weitere Zugabe von Dispergierhilfen wie bspw. mehrwertige Alkohole (wie Glycerin oder Polyethylenglykole) bereits nach einer Stunde fast vollständig.

Weiterhin tritt bei der Herstellung von Apatit-Gelatine-Kompositen eine starke Schaumbildung auf, die sowohl im Labor- als auch im Produktionsmaßstab zu beobachten ist. Diese Schaumbildung beträgt, insbesondere im technischen Maßstab, bis zu 200 % des Volumens der Fällungslauge, so dass entsprechend größere Reaktorgefäße zur Produktion genutzt werden müssen. Darüber hinaus erschwert die Schaumbildung die Zuführung von weiteren Komponenten, insbesondere der Fällungsreagenzien.

Der Erfindung liegt die Aufgabe zugrunde, alternative wirksame Kompositmaterialien bereitzustellen, die die vorstehend genannten Nachteilen des Stands der Technik überwinden.

Bakos et al. beschreiben in "Biomaterials", 20 (1999) Nr. 2, Seiten 191 bis 195, die Verwendung von Kompositen, enthaltend Hydroxyapatit, Collagen und Hyaluronsäure, als Knochen-Implantate. Hierbei wird ein Hydroxyapatit mit Teilchengrößen im Bereich von 40 bis 280 µm eingesetzt.

Die WO02/070030 A betrifft eine Zusammensetzung aus Hyaluronsäure-Derivaten und Hydroxapatit oder anderen Calciumphosphaten, die zur Regeneration von Knochen-Defekten Verwendung finden kann.

Die US-A-4,299,790 beschreibt ein pulverförmiges Gemisch aus Calciumsulfat-Hemihydrat und Xanthan-Gum zur Herstellung von Gips-Figuren. Xanthan-Gum dient hierbei zur Verbesserung der Stärke und des Zusammenhalts der Figuren.

EP-A-0 296 078 offenbart eine Formulierung zur Regeneration von Knochen-Defekten, die neben Calciumsalzen ferner Collagen, Chitosan und Glycosaminoglykane enthält.

Verrier et al. beschreiben in "Journal of Materials Science - Materials in Medicine", Vol. 7 (1996), Nr. 1, Seiten 46 bis 51, ein Kompositmaterial, das Hydroxyapatit neben Collagen und Chondroitin-6-sulfat enthält, als Komponente zur Behandlung von Knochen-Defekten oder Zahn-Implantaten.

Die FR-A-2 585 576 betrifft eine Formulierung aus Collagen-Hydroxyapatit-Glycosaminoglykan zur Regeneration von Knochen-Defekten.

Gegenstand der Erfindung sind demgegenüber Kompositmaterialien umfassend in Wasser schwerlösliche Calciumsalze in Form von nanopartikulären Primärteilchen mit einem mittleren Teilchendurchmesser im Bereich von 10 bis 150 nm, die bei 20°C zu weniger als 0,1 Gew.-% in Wasser löslich sind, und Glucuronsäure-haltige Polysaccharide, ausgewählt aus Xanthan, Welan und Gummi Arabicum, aus Akazien.

Unter Kompositmaterialien werden Verbundstoffe verstanden, welche die vorstehend genannten Komponenten umfassen und mikroskopisch heterogene, makroskopisch aber homogen erscheinende Aggregate darstellen, und in welchen die Primärpartikel der Calciumsalze an das Gerüst des Glucuronsäure-haltigen Polysaccarids assoziiert vorliegen. Der Anteil der Glucuronsäure-haltigen Polysaccharidkomponente in den Kompositmaterialien liegt zwischen 0,1 und 60 Gew.-%, bevorzugt jedoch zwischen 0,5 und 35 Gew.-%, bezogen auf das Gesamtgewicht der Kompositmaterialien.

Unter Primärteilchen werden die Kristallite, d. h. die Einzelkristalle der genannten Calciumsalze verstanden. Als Teilchendurchmesser soll hier der Durchmesser der Teilchen in Richtung ihrer größten Längenausdehnung verstanden werden. Unter dem mittleren Teilchendurchmesser ist ein über die Gesamtmenge des Komposits gemittelter Wert zu verstehen. Die Bestimmung der Teilchendurchmesser kann durch dem Fachmann geläufigen Methoden bestimmt werden, beispielsweise durch die Methode der Transmissionselektronenmikroskopie (TEM).

Vorzugsweise liegen die nanopartikulären Primärteilchen als stäbchenförmige Partikel vor, mit einer Dicke im Bereich von 2 bis 50 nm und einer Länge im Bereich von 10 bis 150 nm. Unter Dicke ist hier der kleinste Durchmesser der Stäbchen zu verstehen, unter Länge ihr größter Durchmesser.

Überraschenderweise wurde gefunden, dass Kompositmaterialien mit kristallinen anorganischen Nanopartikeln erzeugt werden können, in welchen die Nanopartikel eine mikroskopisch deutlich erkennbare kristalline Morphologie aufweisen.

Es wurde weiterhin gefunden, daß die erfindungsgemäßen strukturierten Kompositmaterialien im Gegensatz zum Stand der Technik zu einen besonders effektiven Biomineralisationsprozeß führen. Es wird angenommen, daß dies mit der Nanostruktur des Kompositmaterials und insbesondere der Größe und Morphologie der Calciumsalz-Kristalle zusammenhängt. So wird angenommen, daß die Längsachse der Calciumsalz-Nanopartikel eine Vorzugsrichtung für das weitere Kristallwachstum während der Biomineralisation darstellt.

Unter Glucuronsäure- haltigen Polysacchariden, sind solche Polysaccharide zu verstehen, die aus Glucuronsäure, bevorzugt D-Glucuronsäure, aufgebaut sind. Ein Bestandteil des Kohlenhydratgerüsts wird dabei von Glucuronsäure gebildet. Vorteilhafterweise kann durch die anionischen Carboxylgruppen der Glucuronsäure haltigen Polysaccharide eine besonders gute Wechselwirkung mit dem Calciumsalz erreicht werden, die zu einem besonders stabilen und gleichzeitig besonders gut biomineralisierenden Kompositmaterial führen. Geeignete Polysaccharide sind die Glucuronsäure- haltigen: mikrobiell hergestelltes Xanthan oder Welan oder Gummi Arabicum, welches aus Akazien gewonnen wird.

Ein Vorteil der erfindungsgemäßen Kompositmaterialien ist die besondere Stabilität in wäßrigen Systemen auch ohne Zugabe von Dispergierhilfen wie bspw. mehrwertigen Alkoholen (wie Glycerin oder Polyethylenglykolen).

Weiterhin weisen die erfindungsgemäßen Kompositmaterialien vorteilhafterweise nur eine geringe, bevorzugt im wesentlichen keine Schaumbildung bei der Herstellung auf. Daher werden vorteilhafterweise kleinere Reaktionsgefäße zur Herstellung der Kompositmaterialien benötigt, als es aufgrund einer sehr starken Schaumbildung beispielsweise für Apatit-Gelatine-Komposite notwendig ist. Weiterhin wird die Zugabe der Fällungsreagentien nicht durch die Schaumbildung behindert und der Fällungsprozess somit vereinfacht.

Gemäß einer besonders bevorzugten Ausführungsform weisen die erfindungsgemäßen Calciumsalze eine länglich, insbesondere stäbchen- oder nadelähnliche Form auf. Dies hat den besonderen Vorteil, dass sie der Form der biologischen Apatite (z.B. Knochen- bzw. Dentinapatite) sehr ähnlich sind und daher eine besonders gute Fähigkeit zur Re- und Neomineralisierung aufweisen. Solche Calciumsalze lassen sich z.B. nach dem aus DE 198 58 662 A1 bekannten Verfahren in Form stäbchenförmiger Primärteilchen herstellen.

Als in Wasser schwerlösliches Calciumsalz sollen solche Salze verstanden werden, die bei 20°C zu weniger als 0,1 Gew.-% (1g/I) in Wasser löslich sind. Solche geeigneten Salze sind z.B. Calciumhydroxyphosphat (Ca₅[OH(PO₄)₃]) bzw. Hydroxylapatit, Calciumfluorphosphat . (Ca₅[F(PO₄)₃]) bzw. Fluorapatit, fluordotierter Hydroxylapatit der Zusammensetzung Ca₅(PO₄)₃(OH,F) und Calciumfluorid (CaF₂) bzw. Fluorit oder Flußspat sowie andere Calciumphosphate wie Di-, Tri- oder Tetracalciumphosphat (Ca₂P₂O₇, Ca₃(PO₄)₂, Ca₄P₂O₉, Oxyapatit (Ca₁₀(PO₄)₆O) oder nichtstöchiometrischer Hydroxylapatit (Ca_{5-½ (x+y)} (PO₄)₃₋ₓ (HPO₄)ₓ (OH)_{1-y}). Geeignet sind ebenfalls carbonathaltige Calciumphosphate (z.B. Ca_{5-½ (x+y+z)} (PO₄)_{3-x-z} (HPO₄)ₓ (CO₃)_{z}(OH)_{1-y}), Calciumhydrogenphosphat (z.B. CaH(PO₄)*2 H₂O) und Octacalciumphosphat (z.B. Ca₈H₂(PO₄)*5 H₂O).

Als Calciumsalz kann in den erfindungsgemäßen Kompositmaterialien eines oder auch mehrere Salze im Gemisch, ausgewählt aus der Gruppe von Phosphaten, Fluoriden und Fluorophosphaten, die wahlweise zusätzlich Hydroxyl- und/oder Carbonat-Gruppen enthalten können, im Gemisch enthalten sein. Insbesondere sind die schwer wasserfösliche Calciumsalze ausgewählt aus der Gruppe Hydroxylapatit und Fluorapatit.

Gemäß einer bevorzugten Ausführungsform der vorliegenden Erfindung wird als glucuronsäurehaltiges Polysaccharid Xanthan eingesetzt. Xanthan (auch als Xanthan Gum bezeichnet) ist ein anionisches Heteropolysaccharid, das unter aeroben Bedingungen von Xanthomonas campestris, X. juglandis, Pseudomonas aeruginosa oder Azobacter vinelandii gebildet wird. Es besteht aus einer Hauptkette mit β-1,4 verknüpften Glucose-Bausteinen, bevorzugt D-Glucose, und dreigliedrigen Seitenketten aus D-Mannose, D-Glucuronsäure, Acetat und Pyruvat, die mit jeder zweiten Glucose Einheit verknüpft sind. Die Molmasse beträgt bevorzugt zwischen 1 und 15 x 10⁶ g/mol.

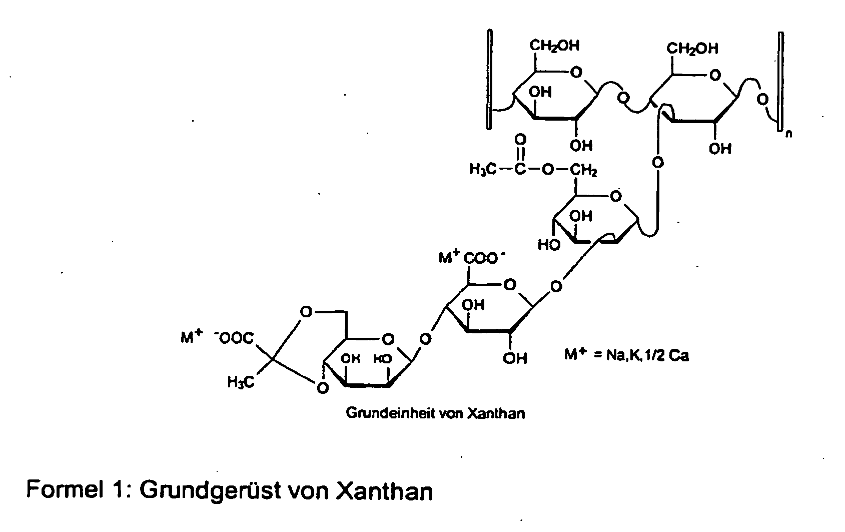

Die Verwendung von Xanthan als Polysaccharidkomponente weist vorteilhafterweise neben einer besonders guten Verarbeitungsfähigkeit, einer besonders hohen Dispersionsstabilität insbesondere von wäßrigen Lösungen auch ohne zusätzliche Dispergierhilfsstoffe auch eine besonders hohe Resistenz gegen mikrobiellen Befall auf.

Weiterhin ist Welan-Gum besonders bevorzugt. Das Welan Gum Grundgerüst is aus D-Glucose, D-Glucuronsäure, D-Glucose und L-Rhamnose Einheiten aufgebaut. Die Seitenketten enthalten jeweils entweder einzelne L-Mannose oder L-Rhamnose Einheiten.

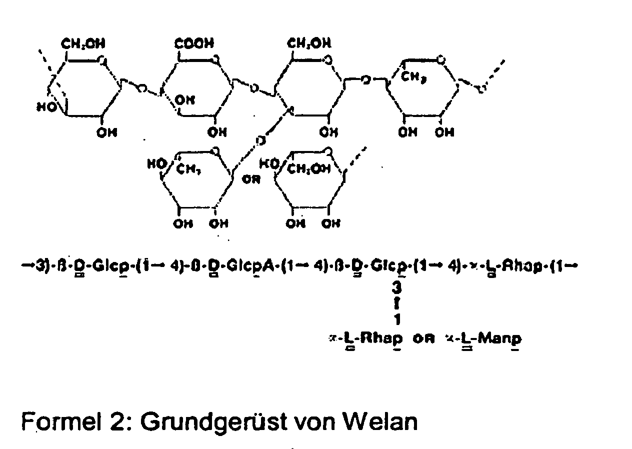

Gemäß einer weiteren Ausführungsform der Erfindung können die in den Kompositmaterialien vorliegenden nanopartikulären Calciumsalz-Primärteilchen von einem oder mehreren Oberflächenmodifikationsmitteln umhüllt sein.

Dadurch kann beispielsweise die Herstellung von Kompositmaterialien in solchen Fällen erleichtert werden, bei welchen sich die nanopartikulären Calciumsalze schwer dispergieren lassen. Das Oberflächenmodifikationsmittel wird an die Oberfläche der Nanopartikel adsorbiert und verändert sie dergestalt, daß die Dispergierbarkeit des Calciumsalzes zunimmt und die Agglomeration der Nanopartikel verhindert wird.

Darüber hinaus kann durch eine Oberfiächenmodifikation die Struktur der Kompositmaterialien sowie die Beladung der Polysaccharidkomponente mit dem nanopartikutären Calciumsalz beeinflußt werden. Auf diese Weise ist es bei der Anwendung der Kompositmaterialien in Remineralisationsprozessen möglich, Einfluß auf den Verlauf und die Geschwindigkeit des Remineralisationsprozesses zu nehmen.

Unter Oberflächenmodifikationsmitteln sind Stoffe zu verstehen, welche an der Oberfläche der feinteiligen Partikel physikalisch anhaften, mit diesen jedoch nicht chemisch reagieren. Die einzelnen an der Oberfläche adsorbierten Moleküle der Oberflächenmodifikationsmittel sind im wesentlichen frei von intermolekularen Bindungen untereinander. Unter Oberflächenmodifikationsmitteln sind insbesondere Dispergiermittel zu verstehen. Dispergiermittel sind dem Fachmann beispielsweise auch unter den Begriffen Emulgatoren, Schutzkolloide, Netzmittel, Detergentien etc. bekannt.

Als Oberflächenmodifikationsmittel kommen beispielsweise Emulgatoren vom Typ der nichtionogenen Tenside aus mindestens einer der folgenden Gruppen in Frage:
Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
C_{12/18}-Fettsäutemono- und -diester von Anlagerungsprodukten von 1 bis 30 Mol Ethylenoxid an Glycerin;
Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
Alkylmono- und -oligoglycoside mit 8 bis 22 Kohlenstoffatomen im Alkylrest und deren ethoxylierte Analoga;
Anlagerungsprodukte von 15 bis 60 Mol Ethylenoxid an Ricinusöl und/oder gehärtetes Rizinusöl;
Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
Anlagerungsprodukte von 2 bis 15 Mol Ethylenoxid an Ricinusöl und/oder gehärtetes Ricinusöl;
Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
Mono-, Di- und Trialkylphosphate sowie Mono-, Di- und/oder Tri-PEGalkylphosphate und deren Salze;
Wollwachsalkohole;
Polysiloxan-Polyalkyl-Polyether-Copolymere bzw. entsprechende Derivate;
Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Rizinusöl stellen bekannte, im Handel erhältliche Produkte dar. Es handelt sich dabei um Homologen-gemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosac-chariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für anionische Emulgatoren sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate wie beispielsweise Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfo-succinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acylglutamate und Acylaspartate, Alkyloligoglucosidsulfate, Proteinfettsäurekondensate (insbesondere pflanzliche Produkte auf Weizenbasis), und Alkyl(ether)phosphate. Sofern die anionischen Tenside Polyglycoletherketten enthalten, können diese eine konventionelle, vorzugsweise jedoch eine eingeengte Homologenverteilung aufweisen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind dies sogenannten Betaine wie die N-Alkyl-N,N-dimethylammoniumglycinate, beispielsweise das Kokosalkyldimethylammoniumglycinat, N-Acylamino-propyl-N,N-dimethylammonium-glycinate, beispielsweise das Kokosacylaminopropyldimethylammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder - Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampho-lytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkyl-gruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind.

In der Regel werden die Oberflächenmodifikationsmittel in einer Konzentration von 0,1 bis 50, vorzugsweise jedoch 1 bis 20 Gew.-%, bezogen auf die Calciumsalze, eingesetzt.

Als Oberflächenmodifikationsmittel bevorzugt geeignet sind vor allem die nichtionischen Tenside in einer Menge von 1 bis 20 Gew.-%, bezogen auf das Gewicht des Calciumsalzes. Als besonders wirksam haben sich die nichtionischen Tenside vom Typ der Alkyl-C₈-C₁₆-(oligo)-glucoside und der Ethoxylate des gehärteten Rizinusöls erwiesen.

Die erfindungsgemäßen Kompositmaterialien werden durch Fällungsreaktionen aus wässrigen Lösungen wasserlöslicher Calciumsalze und wässrigen Lösungen wasserlöslicher Phosphat- und/oder Fluoridsalze hergestellt werden, wobei die Fällung in Gegenwart von der Glucuronsäure haltigen Polysaccharidkomponente durchgeführt wird.

Dies erfolgt vorzugsweise in der Weise, daß die Glucuronsäure-haltige Polysaccharidkomponente in reiner, gelöster oder kolloidaler Form der neutralen wäßrigen Phosphat- und / oder Fluorid-Salzlösung oder der neutralen Lösung des Calciumsalzes vor der Fällungsreaktion beigefügt werden. Alternativ kann die Glucuronsäure- haltige Polysaccharidkomponente in reiner, gelöster oder kolloidaler Form vorgelegt und anschließend nacheinander in beliebiger Reihenfolge der gleichzeitig mit der neutralen Calcium-Salzlösung sowie der neutralen Phosphat- und/oder Fluorid-Salzlösung versetzt werden, wobei die pH-Konstanz, insbesondere zu Zugabe einer Base (beispielsweise Ammoniak) gewährleistet sein muß. Als weitere Möglichkeit können die Komponenten auch direkt in alkalischen Lösungen eingesetzt werden

Bei den erfindungsgemäßen Herstellverfahren kann die Zusammenfügung der einzelnen Komponenten grundsätzlich in allen möglichen Reihenfolgen erfolgen. Als Alkalisierungsmittel wird bevorzugt Ammoniak verwendet.

Eine weitere erfindungsgemäße Variante des Herstellverfahrens besteht darin, daß man die Fällung aus einer sauren Lösung eines wasserlöslichen Calciumsalzes zusammen mit einer stöchiometrischen Menge eines wasserlöslichen Phosphat- und / oder Fluoridsalzes oder aus einer sauren Lösung von Hydroxylapatit mit einem pH-Wert unterhalb von 5, bevorzugt bei einem pH-Wert unterhalb von 3, durch Anheben des pH-Werts mit wäßrigem Alkali oder Ammoniak in Gegenwart der Glucuronsäure- haltigen Polysaccharidkomponente durchführt.

Eine weitere Verfahrensvariante besteht darin, daß man nanopartikuläre Calciumsalze in reiner oder dispergierter Form oder durch Fällungsreaktionen aus wässrigen Lösungen wasserlöslicher Calciumsalze und wässrigen Lösungen wasserlöslicher Phosphat- und/oder Fluoridsalze hergestellte Dispersionen nanopartikulärer Calciumsalze mit der Glucuronsäure-haltigen Polysaccharidkomponente, letztere bevorzugt in gelöster oder dispergierter Form, versetzt, wobei bei der Zugabe eine beliebige Reihenfolge gewählt werden kann.

Bevorzugt wird die Lösung oder Dispersion der Glucuronsäure-haltige Polysaccharidkomponente vorgelegt und eine Dispersion des nanopartikulären Calciumsalzes zugefügt.

Bei den Herstellverfahren kann die entstehende Dispersion des Kompositmaterials nach Bedarf [vgl. Beispiel 1] durch dem Fachmann bekannte Verfahren wie z. B. Filtration oder Zentrifugation vom Lösungsmittel und den übrigen Bestandteilen des Reaktionsgemischs abgetrennt und durch anschließende Trocknung, z. B. durch Gefriertrocknung, in lösungsmittelfreier Form isoliert werden.

Werden bei der Synthese ganz besonders sedimentationsstabile Dispersionen erhalten [vgl. bspw. Beispiel 2] können Zentrifugation und Filtration nicht angewendet werden, weshalb die niedermolekularen Verunreinigungen durch Dialyse abgetrennt werden müssen. Die so gewonnene reinen Dispersionen können dann wiederum durch die oben genannten schonenden Trocknungsverfahren vom Lösungsmittel befreit werden, oder aber durch schonendes Abdestillieren des Wassers im Vakuum eingeengt werden. Ein Verhältnis (in Gew.-%) von Polysaccharidkomponente zu dem schwer wasserlöslichen Calciumsalz ab etwa 0,3:1, und insbesondere größeren Konzentrationen der Polysaccharidkomponente, führt bevorzugter Weise zu solchen ganz besonders stabilen, insbesondere sehr sedimentationsstabilen Dispersionen des erfindungsgemäßen Komposits, bevorzugt auf wäßriger Basis,.

Als Lösungsmittel wird bei allen Herstellungsprozessen bevorzugt Wasser verwendet, jedoch können in einzelnen Schritten der Herstellung auch organische Lösungsmittel wie z. B. Alkohole mit 1 bis 4 C-Atomen oder Glycerin verwendet werden.

Die Herstellung der erfindungsgemäßen Kompositmaterialien, in welchen die Primärpartikel der Calciumsalze oberflächenmodifiziert sind, kann nach analogen Fällungsverfahren wie vorstehend beschrieben erfolgen, wobei jedoch die Fällung der nanopartikulären Calciumsalze oder der Kompositmaterialien in Gegenwart eines oder mehrerer Oberflächenmodifikationsmittel erfolgt.
Bevorzugt wird zunächst durch eine Fällungsreaktion zwischen wäßrigen Lösungen von Calciumsalzen und wäßrigen Lösungen von Phosphat- und / oder Fluoridsalzen in Gegenwart der Oberflächenmodifikationsmittel die oberflächenmodifizierten nanopartikulären Calciumsalze erzeugt. Diese können anschließend von Begleitprodukten des Reaktionsgemischs gereinigt werden, z.B. durch Einengen unter reduziertem Druck und anschließende Dialyse. Durch Abziehen des Lösungsmittels kann zusätzlich eine Dispersion des oberflächenmodifizierten Calciumsalzes mit einem Feststoffanteil nach Wunsch hergestellt werden. Anschließend wird durch Zugabe der Glucuronsäure-haltigen Polysaccharidkomponente in reiner, gelöster oder kolloidaler Form, wobei wiederum die Reihenfolge der Zugabe unkritisch ist, und erforderlichenfalls Nachreaktion bei erhöhter Temperatur, bevorzugt im Bereich zwischen 50 und 100 °C und für eine Dauer von 1 bis 100 Minuten, das Kompositmaterial aus oberflächenbeschichtetem Calciumsalz und Glucuronsäure-haltigen Polysaccharidkomponente gebildet.

Zur Herstellung von Dispersionen oberflächenmodifizierter Calciumsalze können weitere Verfahren herangezogen werden, wie die in der deutschen Anmeldung DE 19858662.0 beschriebenen.

Die erfindungsgemäßen Kompositmaterialien, insbesondere die von Hydroxylapatit, Fluorapatit und Calciumfluorid, eignen sich als remineralisierende Komponente zur Herstellung von Zusammensetzungen zur Reinigung und/oder Pflege der Zähne. Durch die strukturierte Form der Komposite und die Partikelgröße der darin enthaltenen Calciumverbindungen kann die Wirkung einer Festigung des Zahnschmelzes und des Verschlusses von Läsionen und Dentinkanälchen besonders rasch und vollständig erfolgen. Die Zusammensetzungen zur Reinigung und Pflege der Zähne können dabei beispielsweise in Form von Pasten, flüssigen Cremes, Gelen oder Mundspülungen vorliegen. Selbst in flüssigen Zubereitungen verteilen sich die erfindungsgemäßen Kompositmaterialien leicht, bleiben stabil dispergiert und neigen nicht zur Sedimentation.

Eine bevorzugte Ausführungsform sind Zahnpasten mit einem Gehalt an Kieselsäure, Poliermitteln, Feuchthaltemitteln, Bindemitteln und Aromen, die 0,1 - 10 Gew.-% erfindungsgemäße Kompositmaterialien mit nanopartikulären Calciumsalzen aus der Gruppe Hydroxylapatit, Fluorapatit und Calciumfluorid enthalten.

Die Zubereitungen zur Reinigung und Pflege der Zähne können dabei die üblichen Komponenten und Hilfsmittel solcher Zusammensetzungen in den dafür üblichen Mengen enthalten. Für Zahnpasten sind dies z.B.

Putz- und Polierkörper wie z.B. Kreide, Kieselsäuren, Aluminiumhydroxid, Aluminiumsilikate, Calciumpyrophosphat, Dicalciumphosphat, unlösliches Natriummetaphosphat oder Kunstharzpulver
Feuchthaltemittel wie z.B. Glycerin, 1,2-Propylenglycol, Sorbit, Xylit und Polyethylenglycole
Bindemittel und Konsistenzregler, z.B. natürliche und synthetische wasserlösliche Polymere und wasserlösliche Derivate von Naturstoffen, z.B. Celluloseether, Schichtsilikate, feinteilige Kieselsäuren (Aerogel-Kieselsäuren, pyrogene Kieselsäuren)
Aromen, z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen
Süßstoffe wie z.B. Saccharin-Natrium, Natrium-cyclamat, Aspartame, Acesulfan K, Steviosid, Monellin, Glycyrrhicin, Dulcin, Lactose, Maltose oder Fructose Konservierungsmittel und antimikrobielle Stoffe wie z.B. p-Hydroxybenzoesäureester, Natriumsorbat, Triclosan, Hexachlorphen, Phenylsalicylsäureeter, Thymol usw.
Pigmente wie z.B. Titandioxid oder Pigmentfarbstoffe zur Erzeugung farbiger Streifen
Puffersubstanzen z.B. primäre, sekundäre oder tertiäre Alkaliphosphate, Citronensäure/Na-Citrat
wundheilende und entzündungshemmende Wirkstoffe, z.B. Allantoin, Harnstoff, Azulen, Panthenol, Acetylsalicylsäure-Derivate, Pflanzenextrakte, Vitamine, z.B. Retinol oder Tocopherol.

Die erfindungsgemäßen Kompositmaterialien, insbesondere die von Hydroxylapatit und Fluorapatit, können die Biomineralisation in Knochengewebe induzieren oder fördern. Sie eignen sich daher weiterhin als blomineralisierende Komponente zur Herstellung von Zusammensetzungen zur Wiederherstellung oder Neubildung von Knochenmaterial, wie z. B. von Zusammensetzungen zur Behandlung von Knochendefekten und Knochenfakturen sowie zur Förderung des Einwachsens von Implantaten.

Ein weiterer Gegenstand der Erfindung ist eine Süßigkeit, insbesondere ausgewählt aus der Gruppe der Zuckerwaren, enthaltend ein erfindungsgemäßes Kompositmaterial aus einem schwer wasserlöslichen Calciumsalz und einem Glucuronsäure- haltigen Polysaccharid. Zuckerwaren sind eine vielfältige Gruppe von Lebensmitteln, die gemäß der Richtlinie für Zuckerwaren des Bundesverbandes der deutschen Süßwarenindustrie durch Zucker und/oder andere verkehrsübliche Zuckerarten, ggf. Zuckeralkohole, Süßstoffe oder andere süße Zutaten meist einen ausgeprägt süßen Geschmack haben. Zuckerwaren sind auch Füllungs-, Glasur- oder Konfektmassen, sowie Schichten, Überzüge oder Füllungen von Süßwaren oder feinen Backwaren. Zu den Zuckerwaren zählen auch zuckerfreie Zuckerwaren. Bei diesen wird der süße Geschmack durch Zuckeralkohole und/oder Süßstoffe erzielt.

Bevorzugte Zuckerwaren sind insbesondere Kaugummis, Hart- und Weichkaramellen, Gummibonbons, Geleeerzeugnisse, Schaumzuckerwaren, Lakritzwaren, Dragees, Pastillen und kandierte Früchte.

Zur Beschichtung von Implantaten können die erfindungsgemäßen Dispersionen der Kompositmaterialien beispielsweise nach den dem Fachmann bekannten Standardverfahren der Tauchbeschichtung oder des Spritzens aufgebracht werden.

Zur Verwendung als injizierbare Knochenersatzmaterialien können die erfindungsgemäßen Kompositmaterialien mit geeigneten weiteren Stoffen kombiniert werden, wie z. B. Glykosaminoglykanen oder Proteinen, und mit geeigneten Lösungs- und Hilfsmitteln wie z. B. einem verdünnten wäßrigen Phosphatpuffer formuliert werden.

Die folgenden Beispiele sollen den Erfindungsgegenstand näher erläutern:

### Beispiele

### 1. Herstellung von Kompositmaterialien durch Fällungsreaktionen in Gegenwart von Xanthan

Bei 25 °C wird eine Lösung von 44.1 g CaCl₂ ·2 H₂O in 2000 ml entionisiertem Wasser vorgelegt. Unter starkem Rühren werden 10 g Xanthan Gum (Keltrol, Kelko Germany GmbH) zugegeben. Nach etwa 4 h hat sich das Polymer weitgehend gelöst und es liegt eine viskose Flüssigkeit vor. Anschließend werden unter starkem Rühren innerhalb von 1 h 300 mL einer 0.60 M (NH₄)₂HPO₄-Lösung zugetropft, die zuvor auf pH 7 eingestellt wurde. Durch die Zugabe von Ammoniak wird der pH-Wert der Fällungslauge konstant auf 7 gehalten. Die milchige Dispersion wird 21 h bei 25 °C weiter gerührt. Anschließend wird der Feststoffanteil durch Zentrifugieren von der Lösung getrennt. Durch fünffaches Aufschütteln des Rückstandes in demineralisiertem Wasser und anschließende erneutes Zentrifugieren werden die Salze weitgehend ausgewaschen, so dass kein Chlorid mehr nachweisbar ist. Nach der Redispergierung zu einer 2 Gew.-%igen Dispersion des Komposits in Wasser, zeigt diese im Gegensatz zu den entsprechenden Proteinkompositen selbst über einen Zeitraum von über 7 Tagen nur geringfügige Sedimentation.

Mittels Röntgenbeugung kann eindeutig das Vorliegen von nanokristallinem Apatit nachgewiesen werden. Entsprechend können im Transmissionselektronenmikroskop Netzebenen und ausschließlich nadelförmige Morphologien (50-100 x 8 nm) beobachtet werden.

### 2. Herstellung von ganz besonders dispersionsstabilen Kompositmaterialien durch Fällungsreaktionen in Gegenwart von Xanthan

Bei 25 °C wird eine Lösung von 44.1 g CaCl₂ ·2 H₂O in 2000 ml entionisiertem Wasser vorgelegt. Unter starkem Rühren werden 15 g Xanthan Gum zugegeben. Nach etwa 4 h hat sich das Polymer weitgehend gelöst und es liegt eine viskose Flüssigkeit vor. Anschließend werden unter starkem Rühren innerhalb von 1 h 300 mL einer 0.60 M (NH₄)₂HPO₄-Lösung zugetropft, die zuvor auf pH 7 eingestellt wurde. Durch die Zugabe von Ammoniak wird der pH-Wert der Fällungslauge konstant auf 7 gehalten. Die milchige Dispersion wird 21 h bei 25 °C weiter gerührt, dann in Dialyseschläuche gefüllt und so lange dialysiert bis im Außenwasser kein Chlorid mehr nachgewiesen werden kann (Silbernitrat). Die resultierende Dispersion ist gegen Sedimentation über einem Zeitraum von über 7 Tagen vollkommen stabil.

Das erhaltene Material zeigt bei Röntgenbeugungsexperimenten keine Reflexe, dennoch werden im Transmissionselektronenmikroskop Netzebenen und ausschließlich nadelförmige Morphologien (50 -100 x 8 nm) beobachtet.

### 3. Viskositätsmessungen während der Fällung des Xanthan-Apatit-Komposits:

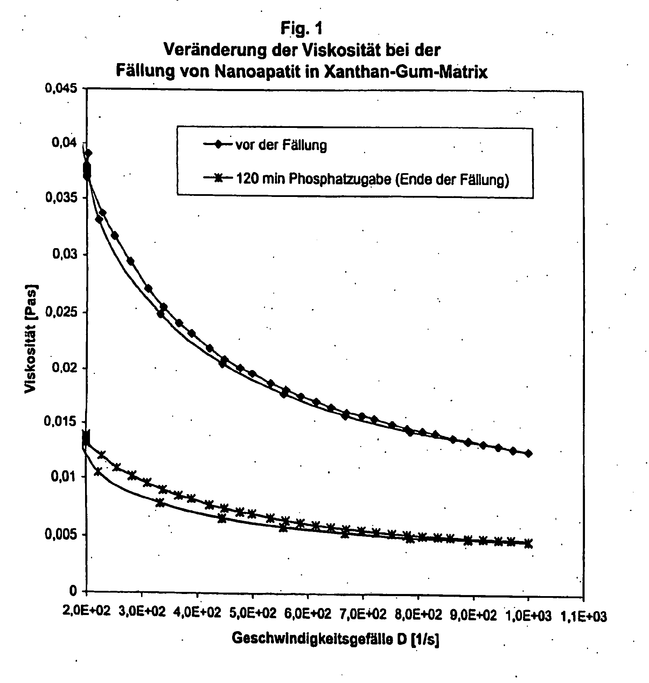

In Fig. 1 ist die Veränderung der Viskosität der Dispersion bei der Ausfällung des Xanthan-Apatit-Komposits nach Beispiel 1 aufgezeigt. Die Viskositäten wurden vor und nach der Ausfällung durch Zutropfen der Phosphatlösung vorgenommen. Vor der Ausfällung befinden sich 0,5 Gew.-% Xanthan und 2 Gew.-% Calciumsalz (gem. Beispiel 1, Konzentrationen beziehen sich auf die Gesamtlösung am Ende der Fällung) in einer wäßrigen Lösung. Die Viskosität wurde mit einem Coutte-Viskosimeter bei einer Temperatur von 25 °C, beim Abbremsen des Zylinders (Geschwindigkeitsgefälle) von 1000 s⁻¹ auf 0 s⁻¹ innerhalb von 5 min bestimmt (Spalt 0,5 mm, Länge des Messkörpers 67,5 mm). Die gemessene Viskositätsentwicklung entsprechen den Werten bei Zugabe einer Mischung aus gemahlenem Apatit (Riedl de Haen, gemahlen, Korngröße >1 µm) 1,5 Gew.-% bezogen auf die Gesamtlösung) und Xanthan (0,5 Gew.-% bezogen auf die Gesamtlösung).

Durch Zutropfen der Phosphatlösung gemäß Beispiel 1 wird das Apatit-Xanthan-Komposit gebildet. Überraschenderweise sinkt die Viskosität von 19,5 vor der Fällung auf 6,6 nach der Fällung (in mPa·s bei D = 500 s⁻¹ beim Abbremsen) vgl. Fig. 1).

### 3. Zahncremes mit Calciumsalz-Kompositmaterialien

**Tabelle 1:**

| **Rezeptur-Beispiele** | **3.1** | **3.2** |
|---|---|---|
| Sident^{®} 8 (Synth. Amorph. Kieselsäure, BET 60 m²/g, Stampfdichte: 350 g/l. DEGUSSA) | 10,0 Gew.-% | 10,0 Gew.-% |
| Sident^{®} 22S (Hydrogelkieselsäure, BET 140 m²/g, Stampfdichte: 100 g/l, DEGUSSA) | 7,0 Gew.-% | 7,0 Gew.-% |
| Sipernat^{®} 320DS | 0,8 Gew.-% | 0,8 Gew.-% |
| Kompositmaterial gemäß Beispiel 1 | 5,0 Gew.-% | 5,0 Gew.-% |
| Polywachs^{®} 1550 (Polyethylenglycol, MG : 1550, Erweichungspunkt 45 - 50° C, RWE / DEA) | 2,0 Gew.-% | 2,0 Gew.-% |
| Texapon^{®} K 1296 (Natrium-Laurylsulfat-Pulver, Cognis Deutschland GmbH) | 1,5 Gew.-% | 1,5 Gew.-% |
| Titandioxid | 1,0 Gew.-% | 1,0 Gew.-% |
| Cekol^{®} 500 T (Natrium-Carboxymethylcellulose, Viskosität (2 %ig in Wasser, Brookfield LVF, 20° C) : 350-700 mPa·s, Nordmann-Rassmann) | 1,0 Gew.-% | 1,0 Gew.-% |
| Na-Fluorid | 0,33 Gew.-% | 0,33 Gew.-% |
| Na-Benzoat | 0,25 Gew.-% | 0,25 Gew.-% |
| Aroma | 1,0 Gew.-% | 1,0 Gew.-% |
| Tagat^{®} S (Polyoxyethylen-(20)-glycerylmonostearat, Tego Cosmetics, Goldschmidt) | 0,2 Gew.-% | - |
| Na-Saccharinat | 0,15 Gew.-% | 0,15 Gew.-% |
| Tri-Natriumphosphat | 0,10 Gew.-% | 0,10 Gew.-% |
| Sorbit (70 %ig in Wasser) | 31,0 Gew.-% | 31,0 Gew.-% |
| Wasser | ad 100 Gew.-% | ad 100 Gew.-% |

### 4. Zahngele mit Calciumsalz-Kompositmaterialien

**Tabelle 2: Gel**

| | Gel ohne Fluorid | Gel mit Fluorid |
|---|---|---|
| Carboxymethylcellulose, Na-Salz Blanose^{®} CMC 9M31XF (Hercules) | 1,25g | 1,25g |
| Apatit-Xanthan-Komposit gemäß Beispiel 1 5,90 % in H₂O dispergiert | 2,55g | 2,55g |
| Glycerin, wasserfrei (Merck) | 69g | 69g |
| Maltitsirup (HDS-Chemie, Österreich) | 6,3g | 6,3g |
| Deionisiertes Wasser | 51,0g | 51,0g |
| Natriumfluorid (Merck) | | 0,45g |
| D-Sorbit, 97 %; (Aldrich) | 94g | 94g |
| Xylitol (Sigma) | 11,1g | 11,1g |
| Kieselsäure, gefällt Sipemat^{®} 320 DS (Degussa AG) | 11,75g | 11,75g |
| Lecithin (ACROS organics) | 0,25g | 0,25g |
| Optamint^{®} (Haarmann & Reimer) | 0,25g | 0,25g |
| Konservierungsmittel Paraben K: Lösung von Ethyl-4-hydroxybenzoat (ACROS | 0,29g | 0,29g |
| Organics) | 0,16g | 0,16g |
| Propyl-4-hydroxybenzoat (ACROS | 2,45g | 2,45g |
| Organics) | | |
| In Benzylalkohol (ACROS | | |
| Organics) | | |

### 5. pH-Messungen in künstlichem Speichel:

### 5.1 Methode:

Die Zusammensetzung, die das schwer wasserlöslichen Calciumsalz oder dessen Komposite enthält, wird in eine Salzlösung gegeben, deren Gehalt an anorganischen Salzen demjenigen von Körperflüssigkeiten wie Speichel, Blut oder Plasma entspricht (Simulated Body Fluid, SBF) und die dementsprechend bezogen auf die Ausfällung von Calciumphosphat übersättigt ist. Solche Zusammensetzungen können als Modell für Körperflüssigkeiten eingesetzt werden, wie bereits in Liu et. al, Cells and Materials (1997), 7, S. 41-51) beschrieben wurde.

Für die vorliegende Untersuchung wurde ein SBF ("Simulated Body Fluid") verwendet, die aus einer wässrigen Lösung folgender Salze besteht:

| | | | |
|---|---|---|---|
| Na⁺ | 14 mM | PO₄³⁻ | 4,7mM |
| K⁺ | 21 mM | Cl⁻ | 30 mM |
| Ca²⁺ | 1,8mM | | |

Bei 37°C wurden je 100 mg einer 2-prozentigen Dispersion nach Beispiel 2 in 25 ml des SBF gelöst und die anschließende pH-Wert-Veränderung mittels einer pH-Elektrode (Inlab 410, Mettler Toledo; Meßgerät: Consort, Multi Parameter Analyzer C833) verfolgt.

### 5.2. Messergebnisse:

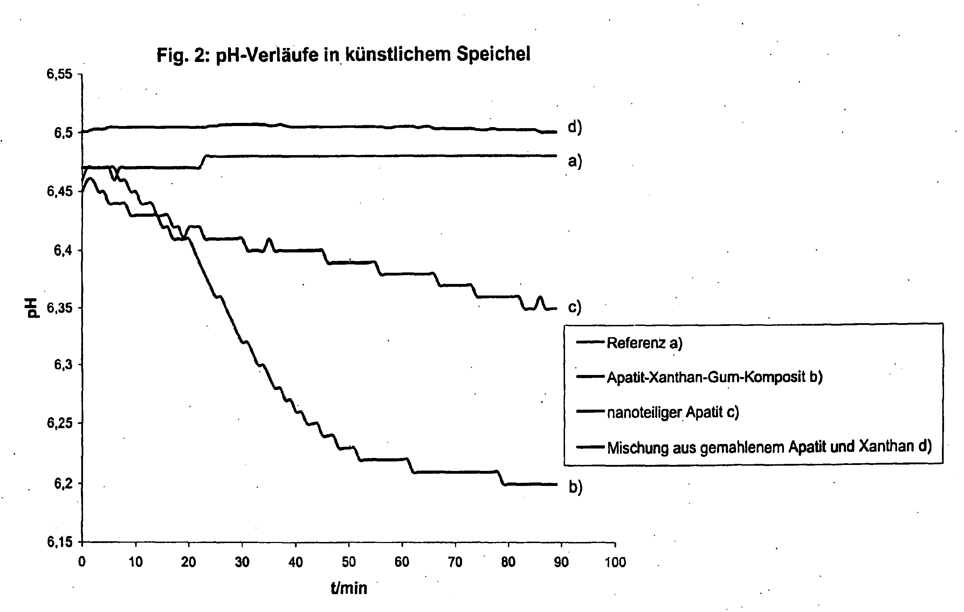

In Fig. 2 sind die pH-Wert-Verläufe zwischen 0 min und 90 min nach Zugabe des Komposits in SBF gezeigt. Der Speichel ohne Zugabe von Material (Referenzwert a) sowie die Zugabe eines Gemisches aus gemahlenem Apatit und Xanthan (b) ergibt keine pH-Wertveränderungen, während das nanoskalige Apatit (c) bereits eine Senkung des pH-Wertes zeigt. Eine besonders starke pH-Wertveränderung ist durch Zugabe des Apatit-Xanthan-Komposits (b) zu beobachten.

Die zu beobachtenden pH-Wertveränderungen sind mit einer durch das schwer wasserlösliche Calciumsalz oder dessen Komposite induzierten Calciumphosphat-Fällung aus dem künstlichen Speichel zu erklären, die entsprechend der folgenden Gleichung, anhand des abfallenden pH-Wertes in der "SBF" gemessen wird (vgl. auch T. Aoba, E.E. Moreno, J. Dent. Res. 63 (1984) 874-880).

10 CaCl₂ + 6 Na₂HPO₄ + 2 H₂O → Ca₁₀(OH)₂(PO₄)₆ + 12 NaCl + 8 HCl

Die Wirkungsweise der schwerlöslichen Calciumsalz-Komposite mit Glucuronsäure- haltigen Polysacchariden liegt (ohne auf diese Theorie beschränkt sein zu wollen) aufgrund der Ergebnisse nicht nur in der Bereitstellung von Calcium- und/oder Phosphationen zum Einbau in die Dentinkanälchen und den Zahnschmelz. Darüber hinaus ist das schwerlösliche Calciumsalz insbesondere in der Lage, aus den im natürlichen Speichel des Menschen in übersättigten Konzentrationen vorliegenden Calcium- und Phosphationen abzuscheiden (vgl. Fig. 2). Diese Nukleationswirkung führt so zur einem Aufbau von neuem Zahnmaterial (Neomineralisierung), insbesondere des Zahnschmelzes und/oder des Dentins, aus den körpereigenen Reservoirs. Gegenüber dem nanoskaligen Calciumsalz alleine weist das Kompositmaterial eine besonders starke nukleierende Wirkung auf. Die bloße Mischung von Xanthan und schwerlöslichem Calciumsalz stellt dagegen kein Kompositstruktur dar, sie wirkt nicht nukleierend, wie in Fig.2 (b) zu beobachten ist.

Vorteilhafterweise können daher die zuzugebenden Mengen an schwer wasserlöslichem Calciumsalz klein gehalten werden, ohne dass die Re- und insbesondere Neomineralisierungseffekte sich abschwächen. Das partikuläre Calciumsalz kann also als Kristallisationskeim für die im natürlichen Speichel vorkommenden Calcium- und Phosphationen wirken.

### 6. Untersuchungen zur Schaumbildung

### 6.1 Schaumbildung bei der Herstellung des Apatit-Xanthan-Komposits

Das Apatit-Xanthan-Komposit wird wie in Beispiel 1 beschrieben hergestellt. Gerührt wird bei der Herstellung mit einem dreiflügeligen Propellerrührer von 10 cm Durchmesser bei 690 U/min.

Bei der Herstellung wird während keiner Phase der Herstellung des Kompositmaterials eine Schaumbildung beobachtet. Lediglich vereinzelte instabile Bläschen finden sich auf der Oberfläche der Fällungslauge.

### 6.2 Schaumbildung bei der Herstellung des Apatit-Gelatine-Komposits

In diesem Vergleichsversuch werden 35 g Gelatine (Typ A, DGF-Stoess, Eberbach) bei 50 °C in 350 ml demineralisierten Wasser gelöst. Diese Lösung wird mit der wie oben angegeben hergestellten Calciumchloridlösung vereinigt und bei 25 °C gerührt. Der pH-Wert wird auf pH 7 eingestellt.

Die weitere Herstellung erfolgt analog zu der in Beispiel 1 angegebenen Weise, allerdings wird die Phosphatlösung-innerhalb von 2 h statt 1 h zugegeben. Gerührt wird mit einem dreiflügeligen Propellerrührer von 10 cm Durchmesser bei 690 U/min.

Während der Herstellung des Kompositmaterials ist eine starke Schaumbildung zu beobachten, die mit der Phosphatzugabe einsetzt und über den gesamten Fällungszeitraum bis zum Ende der Reifungszeit anhält. Die Phosphatzugabe wird durch die Schaumbildung erschwert und daher in einem Zeitraum von zwei Stunden durchgeführt. Am Ende der Fällung beträgt das zusätzliche Schaumvolumen über der Fällungslauge 100 % des Volumen der Fällungslauge. Der entstehende Schaum ist gegen Zerfall über einen Zeitraum von 12 h stabil und kann nicht durch Rühren zerstört werden.

## Patentansprüche

1. Kompositmaterialien umfassend in Wasser schwerlösliche Calciumsalze in Form von nanopartikulären Primärteilchen mit einem mittleren Teilchendurchmesser im Bereich von 10 bis 150 nm, die bei 20°C zu weniger als 0,1 Gew.-% in Wasser löslich sind, und Glucuronsäure-haltige Polysaccharide, ausgewählt aus Xanthan, Welan und Gummi Arabikum aus Akazien.

2. Kompositmaterialien nach Anspruch 1, **dadurch gekennzeichnet, daß** die in Wasser schwerlöslichen Calciumsalze in Form von stäbchenförmigen Primärteilchen vorliegen.

3. Kompositmaterialien nach Anspruch 2, **dadurch gekennzeichnet, dass** die stäbchenförmigen Primärteilchen mit einer Dicke im Bereich von 2 bis 50 nm und einer Länge im Bereich von 10 bis 150 nm vorliegen.

4. Kompositmaterialien nach einem der Ansprüche 1 bis 3, **dadurch gekennzeichnet, daß** die in Wasser schwerlöslichen Calciumsalze ausgewählt sind aus Phosphaten, Fluoriden und Fluorophosphaten, die wahlweise zusätzlich Hydroxyl- und/oder Carbonat-Gruppen enthalten können.

5. Kompositmaterialien nach einem der Ansprüche 1 bis 4, **dadurch gekennzeichnet, daß** das Calciumsalz ausgewählt ist aus der Gruppe Hydroxylapatit und Fluorapatit.

6. Kompositmaterialien nach einem der Ansprüche 1 bis 5, **dadurch gekennzeichnet, daß** als Glucuronsäure-haltiges Polysaccharid Xanthan eingesetzt wird.

7. Kompositmaterialien nach einem der Ansprüche 1 bis 6, **dadurch gekennzeichnet, daß** der Anteil der im Kompositmaterial enthaltenen Glucuronsäure-haltigen Polysaccharidkomponente zwischen 0,5 und 35 Gew.-%, bezogen auf das Gesamtgewicht des Kompositmaterials, beträgt.

8. Kompositmaterialien nach einem der Ansprüche 1 bis 7, **dadurch gekennzeichnet, daß** die als nanopartikuläre Primärpartikel vorliegenden Calciumsalze von einem oder mehreren Oberflächenmodifikationsmitteln umhüllt sind.

9. Verfahren zur Herstellung von Kompositmaterialien nach einem der Ansprüche 1 bis 8 durch Fällungsreaktionen aus wäßrigen Lösungen wasserlöslicher Calciumsalze und wäßrigen Lösungen wasserlöslicher Phosphat- und/oder Fluoridsalze, **dadurch gekennzeichnet, daß** die Fällung in Gegenwart der Glucuronsäure-haltigen Polysaccharidkomponente durchgeführt wird.

10. Verfahren zur Herstellung von Kompositmaterialien nach Anspruch 9 durch Fällung aus einer sauren Lösung eines wasserlöslichen Calciumsalzes und einer stöchiometrischen Menge eines wasserlöslichen Phosphat- und / oder Fluoridsalzes mit einem pH-Wert unterhalb von 3 durch Anheben des pH-Wertes mit wäßrigen Alkalien oder Ammoniak in Gegenwart der Glucuronsäure-haltigen Polysaccharidkomponente.

11. Verwendung der Kompositmaterialien nach einem der Ansprüche 1 bis 8 als remineralisierende Komponente zur Herstellung von Zusammensetzungen zur Reinigung und/oder Pflege der Zähne.

12. Verwendung der Kompositmaterialien nach einem der Ansprüche 1 bis 8 zur Beschichtung von Implantaten.

13. Zahn- und/oder Mundpflegemittel mit einem Gehalt an Kompositmaterialien nach einem der Ansprüche 1 bis 8.

14. Süßigkeit mit einem Gehalt an Kompositmaterialien nach einem der Ansprüche 1 bis 8.

15. Zusammensetzungen zur Induktion der Förderung der Neubildung von Knochengewebe mit einem Gehalt an Kompositmaterialien nach einem der Ansprüche 1 bis 8.

## Claims

1. Composite materials comprising calcium salts hardly soluble in water in the form of nanoparticulate primary particles with an average particle diameter in the range from 10 to 150 nm, which are soluble in water at 20°C in an amount less than 0.1 % by weight, and polysaccharides containing glucuronic acid, selected from xanthan, wellan gum and gum arabic from acacia.

2. The composition materials according to claim 1, **characterized in that** the calcium salts hardly soluble in water exist as rod-like primary particles.

3. The composition materials according to claim 2, **characterized in that** the rod-like primary particles exist with a thickness in the range from 2 to 150 nm and with a length in the range from 10 to 150 nm.

4. The composition materials according to any of claims 1 to 3, **characterized in that** the calcium salts hardly soluble in water are selected from phosphates, fluorides and fluorophosphates, which may further contain optionally hydroxyl and/or carbonate groups.

5. The composition materials according to any of claims 1 to 4 **characterized in that** the calcium salt is selected from the group of hydroxylapatite and fluorapatite.

6. The composition materials according to any of claims 1 to 5, **characterized in that** xanthan is applied as a polysaccharide containing glucuronic acid.

7. The composition materials according to any of claims 1 to 6, **characterized in that** the proportion of the polysaccharide component containing glucuronic acid contained in the composite material is comprised between 0.5 and 35% by weight, based on the total weight of the composite material.

8. The composite materials according to any of claims 1 to 7, **characterized in that** the calcium salts existing as nanoparticulate primary particles are coated with one or more surface modifying agents.

9. A method for preparing composite materials according to any of claims 1 to 8 by precipitation reactions from aqueous solutions of water-soluble calcium salts and aqueous solutions of water-soluble phosphate and/or fluoride salts, **characterized in that** the precipitation is carried out in the presence of the polysaccharide component containing glucuronic acid.

10. The method for preparing composite materials according to claim 9 by precipitation from an acid solution of a water-soluble calcium salt and from a stoichiometric amount of a water-soluble phosphate and/or fluoride salt with a pH value below 3 by raising the pH value with aqueous alkalis or ammonia in the presence of the polysaccharide component containing glucuronic acid.

11. The use of the composite materials according to any of claims 1 to 8 as a re-mineralizing component for preparing compositions for the cleaning and/or care of teeth.

12. The use of composite materials according to any of claims 1 to 8 for coating implants.

13. A dental and/or buccal care agent with a content of composite materials according to any of claims 1 to 8.

14. A sweet with a content of composite materials according to any of claims 1 to 8.

15. compositions for inducing or promoting new formation of bone tissue, with a content of composite materials according to any of claims 1 to 8.

## Revendications

1. Matériaux composites comprenant des sels de calcium difficilement solubles dans l'eau sous la forme de particules primaires nanoparticulaires possédant un diamètre moyen de particule dans la plage de 10 à 150 nm, qui sont solubles dans l'eau à une température de 20 °C à concurrence de moins de 0,1 % en poids, et des polysaccharides contenant de l'acide glucuronique, choisis parmi le xanthane, la gomme Welan et la gomme arabique d'acacia.

2. Matériaux composites selon la revendication 1, **caractérisés en ce que** les sels de calcium difficilement solubles dans l'eau sont présents sous la forme de particules primaires en forme de bâtonnets.

3. Matériaux composites selon la revendication 2, **caractérisés en ce que** les particules primaires en forme de bâtonnets sont présentes avec une épaisseur dans la plage de 2 à 50 nm et une longueur dans la plage de 10 à 150 nm.

4. Matériaux composites selon l'une quelconque des revendications 1 à 3, **caractérisés en ce que** les sels de calcium difficilement solubles dans l'eau sont choisis parmi des phosphatés, des fluorures et des fluorophosphates qui peuvent contenir en outre de manière facultative des groupes hydroxyle et/ou des groupes carbonates.

5. Matériaux composites selon l'une quelconque des revendications 1 à 4, **caractérisés en ce que** le sel de calcium est choisi parmi le groupe de l'hydroxylapatite et de la fluorapatite.

6. Matériaux composites selon l'une quelconque des revendications 1 à 5, **caractérisés en ce que** l'on met en oeuvre du xanthane à titre de polysaccharide contenant de l'acide glucuronique.

7. Matériaux composites selon l'une quelconque des revendications 1 à 6, **caractérisés en ce que** la fraction du composant de polysaccharide contenant de l'acide glucuronique, contenue dans le matériau composite, s'élève entre 0,5 et 35 % en poids, rapportés au poids total du matériau composite.

8. Matériaux composites selon l'une quelconque des revendications 1 à 7, **caractérisés en ce que** les sels de calcium qui sont présents sous la forme de particules primaires nanoparticulaires sont enrobés par un ou plusieurs agents de modification superficiels.

9. Procédé pour la préparation de matériaux composites selon l'une quelconque des revendications 1 à 8, via des réactions de précipitation à partir de solutions aqueuses de sels de calcium solubles dans l'eau et de solutions aqueuses de sels phosphate et/ou fluorure solubles dans l'eau, **caractérisés en ce que** la précipitation est mise en oeuvre en présence du composant de polysaccharide contenant de l'acide glucuronique.

10. Procédé pour la préparation de matériaux composites selon la revendication 9, par précipitation à partir d'une solution acide d'un sel de calcium soluble dans l'eau et d'une quantité stoechiométrique d'un sel phosphate et/ou fluorure soluble dans l'eau possédant une valeur de pH inférieure à 3, par élévation de la valeur de pH avec des alcalis aqueux ou de l'ammoniac en présence du composant de polysaccharide contenant de l'acide glucuronique.

11. Utilisation des matériaux composites selon l'une quelconque des revendications 1 à 8 à titre de composant de reminéralisation pour la préparation de compositions destinées au nettoyage et/ou aux soins des dents.

12. Utilisation des matériaux composites selon l'une quelconque des revendications 1 à 8, pour l'enduction d'implants.

13. Agent de soins pour les dents et/ou pour la bouche possédant une teneur en matériaux composites selon l'une quelconque des revendications 1 à 8.

14. Sucrerie possédant une teneur en matériaux composites selon l'une quelconque des revendications 1 à 8.

15. Compositions pour induire ou favoriser la néoformation de tissu osseux, possédant une teneur en matériaux composites selon l'une quelconque des revendications 1 à 8.
